(19) Europäisches Patentamt
European Patent Office
Office européen des brevets

(11) EP 0 530 671 B1

(12) **EUROPÄISCHE PATENTSCHRIFT**

(45) Veröffentlichungstag und Bekanntmachung des Hinweises auf die Patenterteilung:
**08.10.1997 Patentblatt 1997/41**

(51) Int Cl.[6]: **C12P 17/18**, C07D 493/02, C12P 41/00, C12R 1/39 // (C12P17/18, C12R1:39)

(21) Anmeldenummer: **92114587.6**

(22) Anmeldetag: **27.08.1992**

(54) **Verfahren zur enzymatischen Herstellung isomerenreiner Isosorbid-2- und 5-monoester sowie deren Überführung in Isosorbid-2- und 5-nitrat**

Process for the enzymatic preparation of pure isosorbide-2 or 5-monoester-isomers and conversion to isosorbide-2- and 5-nitrate

Procédé de préparation enzymatique d'isomères purs de l'isosorbide-2- ou 5-monoester et leur conversion en isosorbide-2- et 5-nitrate

(84) Benannte Vertragsstaaten:
**AT BE CH DE DK ES FR GB GR IE IT LI LU NL PT SE**

(30) Priorität: **02.09.1991 DE 4129093**

(43) Veröffentlichungstag der Anmeldung:
**10.03.1993 Patentblatt 1993/10**

(73) Patentinhaber: **BOEHRINGER MANNHEIM GMBH**
**68298 Mannheim (DE)**

(72) Erfinder:
- **Schneider, Manfred, Prof. Dr.**
  **W-5600 Wuppertal 1 (DE)**
- **Seemayer, Robert, Dr.**
  **W-5600 Wuppertal 12 (DE)**

(56) Entgegenhaltungen:
**EP-A- 0 057 847** **EP-A- 0 067 964**
**EP-A- 0 254 243** **US-A- 5 032 523**

- **TETRAHEDRON ASYMETRY Bd. 3, Nr. 9 , September 1992 Seiten 1123 - 1125 SEEMAYER ET AL. 'enzymatic preparation of isomerically pure 1,4-3,6-dianhydro-D-glucitol monoacetates- precursors for isoglucitol-2 and 5-mononitrates'**
- **J. BIOCHEM. Bd. 104, Nr. 5 , November 1988 Seiten 681 - 682 NISHIO ET AL. 'Enzymatic transesterification with the lipase from Pseudomonas fragi 22-39 B in a non-aqueous reaction system'**
- **J. AM. CHEM. SOC. Bd. 110, Nr. 21 , 1988 Seiten 7200 - 7205 WANG ET AL. 'Lipase-catalyzed irreversible transesterifications using enol esters as acylating agents : preparative enantio- and regioselective syntheses of alcohols, glycerol derivatives, sugars, and organometallics'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 530 671 B1

## Beschreibung

Die Erfindung betrifft ein enzymatisches Verfahren zur Herstellung enantiomerenreiner und isomerenreiner Verbindungen der allgemeinen Formel I

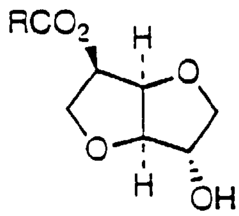

I

wobei R = H und Alkyl von C1 - C17, bevorzugt gegebenenfalls substitutiertes oder halogeniertes Alkyl bedeutet.
sowie isomerenreinen Verbindungen der allgemeinen Formel II

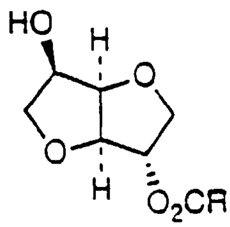

II

wobei R = H und Alkyl von C1 - C17, bevorzugt gegebenenfalls substitutiertes oder halogeniertes Alkyl bedeutet.
Bei diesem Verfahren wird Isosorbid (1,4:3,6-Dianhydro-D-sorbit) der Formel III

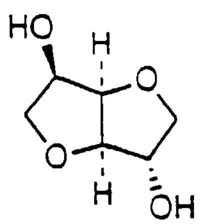

III

durch regioselektive, enzymatische Veresterung selektiv in isomerenreine Verbindungen der allgemeinen Formel I überführt.
Alternativ dazu wird ein Diester von Isosorbid der allgemeinen Formel IV

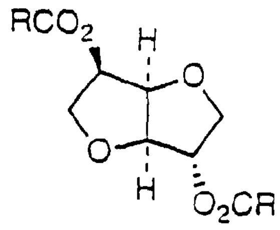

IV

wobei R = H und Alkyl von C1 - C17, bevorzugt gegebenenfalls substituiertes oder halogeniertes Alkyl bedeutet,
durch enzymatische Hydrolyse oder Alkoholyse selektiv in isomerenreine Verbindungen der allgemeinen Formel II überführt.
Die dabei praktisch quantitativ erhaltenen, isomerenreinen Verbindungen der allgemeinen Formein I und II sind Ausgangsmaterialien für die Herstellung der isomerenreinen, sowie enantiomerenreinen Pharmaka Isosorbid-2-nitrat der Formel V, sowie von Isosorbid-5-nitrat der Formel VI.

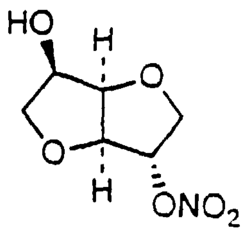

V

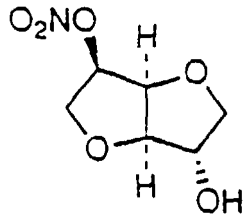

VI

Dazu werden Verbindungen der allgemeinen Formel I zunächst nitriert und danach der Acylrest abgespalten. Man erhält dabei Isosorbid-2-nitrat der Formel V. Ausgehend von Verbindungen der allgemeinen Formel II erhält man auf analoge Weise Isosorbid-2-nitrat der Formel VI.
Beide Verbindungen der Formeln V und VI sind wichtige Therapeutika zur Behandlung koronarer Erkrankungen, wie z.B. Angina pectoris.

Organische Nitrate z.B. Isosorbid-2,5-dinitrat (ISD) werden seit langem zur Therapie koronarer Erkrankungen, wie z.B. von Angina pectoris eingesetzt. Bei Metabolisierungsstudien dieser Verbindungen im Organismus wurde nachgewiesen, daß Isosorbid-2-mononitrat und Isosorbid-5-mononitrat gleichartige Wirkungen wie ISD besitzen [z.B. R.L. Wendt. *J. Pharmacol. Exp. Ther.* **180**, 732 (1971)], wobei die Verabreichung der Mononitrate gegenüber ISD therapeutische Vorteile hat. Diese Verbindungen zeichnen sich vor allem durch höhere Bioverfügbarkeit und längere Halbwertszeit aus.
Einer praktischen Anwendung von Isosorbid-2-nitrat und Isosorbid-5-nitrat stand bislang die aufwendige und meist wenig selektive Synthese dieser Verbindungen im Wege.

Die Herstellung dieser Verbindungen ist in einer Reihe von Veröffentlichungen und Patenten beschrieben [I.G. Czizmadia, L.D. Hayward, *Photochem. Photobiol.* **4**, 657 (1965); M. Anteunis et al., *Org. Magnetic Resonance* **3**, 363 (1971); D.L. Hayward et al., *Can. J. Chem.* **45**, 2191 (1967); DE 2 751 934; US Patent 4 065 488].
Mit keinem dieser beschriebenen Verfahren gelingt die Herstellung von isomerenreinem Isosorbid-2-nitrat oder Isosorbid-5-nitrat ohne aufwendige Trennverfahren wie z.B. Chromatographie, Extraktion, Umkristallisation, Derivatisierung etc.
Derartige Reinigungsschritte sind stets mit beträchtlichen Ausbeuteverlusten verbunden, die eine wirtschaftliche Herstellung dieser Pharmaka unmöglich machen.
Eine selektivere Methode zur Herstellung von Isosorbid-2-nitrat wird in DOS 2 903 983 beschrieben. Dabei wird Isomannid zunächst mittels eines Trifluormethylmethansulfonsäure-halogenids in Anwesenheit eines Säurefängers oder aber mit Trifluormethansulfonsäureanhydrid umgesetzt. Reaktion der so bevorzugt erhaltenen Isomannid-2-trifluormethansulfonsäureester mit Alkali-, Erdalkali- oder organischen Nitraten liefert unter Konfigurationsumkehr am Kohlenstoff C(2) des Ringsystems das gewünschte Isosorbid-2-nitrat.

Problematisch für eine technische Anwendung sind die verhältnismäßig schlechten Ausbeuten. Gegen eine technische Anwendung sprechen auch die relativ teuren Reagenzien und das schwer zugängliche Isomannid.
Wird die oben beschriebene Inversion dagegen zunächst mit Benzoesäure oder Salzen der Benzoesäure durchgeführt, danach nitriert und die Estergruppe abgespalten, so erhält man nach DOS 2 903 927 Isorbid-5-nitrat. Wiederum wird der teure Isomannid verwendet.
Ein weiterer Weg zu Isosorbid-2-nitrat, ausgehend von Isosorbid wird in DOS 3 124 410 beschrieben [s. auch P. Stoss et al., *Synthesis* **1987**, 174]. Dabei wird durch den wahlweisen Einsatz von Metallkatalysatoren, z. B. Bleitetraacetat eine regioselektive Acylierung von Isosorbid durch Carbonsäureanhydride in der 5-Position erreicht, während die unkatalysierte Reaktion bevorzugt zum Isosorbid-2-ester führt. Die so erhaltenen Produkte werden danach nitriert und die Estergruppe zur Herstellung von Isosorbid-2-nitrat bzw. Isosorbid-5-nitrat. durch Umesterung abgespalten. Nachteilig erweist sich bei diesem Verfahren die Verwendung toxischer Metallsalze in einer Synthese therapeutisch anzuwendender Verbindungen. Die Reaktionen sind zwar regioselektiv, aber nicht regiospezifisch. Auch erscheint die Kontrolle der Reaktionsbedingungen in Richtung der gewünschten Acylierungsprodukte durchaus nicht trivial. Die Herstellung von Isosorbid-2-nitrat bzw. Isosorbid-5-nitrat durch Nitrierung eines Isosorbid-5-acylates bzw. Isosorbid-2-acylates sowie anschließende Umwandlung durch Hydrolyse bzw. Umesterung ist ebenfalls beschrieben in EP 0 067 964 und EP 0 057 847.

Eine enzymatische Methode zur Herstellung der isomerenreinen Isosorbid-2- oder 5-monoester, Schlüsselverbindungen bei der Synthese von Isosorbid-5- oder 2-nitrat, wurde bislang nicht beschrieben.

Zusammenfassend läßt sich feststellen, daß alle bislang bekannten Verfahren zur Herstellung von Isosorbid-2-nitrat und Isosorbid-5-nitrat schwerwiegende methodische oder ökonomische Nachteile haben.
Benötigt wird eine hoch selektive, einfach und kostengünstige durchführbare Methode zur Herstellung von Isosorbid-5 und 2-monoestern der allgemeinen Formeln I und II. Es sollte darüber hinaus ein Verfahren sein, mit dem - ausgehend von einfach und preiswert zugänglichem Isosorbid - beide Verbindungen der allgemeinen Formeln I und II wahlweise, isomerenrein, mit hohen Ausbeuten und unter Vermeidung aufwendiger Reinigungsschritte erhalten werden können. Damit wäre auch die Herstellung der isomerenreinen Pharmaka Isosorbid-2-nitrat und Isosorbid-5-nitrat auf einfache und kostengünstige Weise möglich.

Die vorliegende Erfindung beschreibt ein derartiges Verfahren mit dem durch

a) enzymatische Veresterung von Isosorbid (III) Isosorbid-5-monoester (I) und durch enzymatische Hydrolyse oder Alkoholyse von Isosorbid-2,5-diestern (IV) Isosorbid-2-monoester (II) selektiv, isomerenrein und mit hohen Ausbeuten erhalten werden, die sich chemisch in
b) Isosorbid-2-nitrat (V) und Isosorbid-5-nitrat (VI) überführen lassen. Die Erfindung ist in den Ansprüchen 1 - 15 und im folgenden ausführlich beschrieben.

Enzyme haben sich in den letzten Jahren aufgrund ihrer hochselektiven Eigenschaften als attraktive Reagenzien in der organischen Synthese zunehmend bewährt. Dies gilt insbesondere für Hydrolasen (Esterasen, Lipasen, Proteasen), die vielfach zur Herstellung enantiomerenreiner Hydroxyverbindungen durch enantioselektive Hydrolyse oder Veresterung eingesetzt wurden. So wurden beispielsweise Lipasen für irreversible Umesterungen verwendet (US 5,032,523; Wang et al., J. Am. Chem. Soc. 110, 1988, 7200-7205).
Es wurde nun überraschend gefunden, daß sich derartige Esterhydrolasen auch hervorragend zur regioselektiven Differenzierung der 2- und 5-Hydroxygruppen in dem bereits enantiomerenreinen Isosorbid (III) eignen und dadurch wahlweise die selektive Herstellung der isomerenreinen Isosorbid-5-monoester (I) oder Isosorbid-2-monoester (II) gestatten.
Als Ausgangsmaterialien für die im folgenden beschriebenen Reaktionen dient ausschließlich kommerziell verfügbares Isosorbid, sowie das daraus durch Acylierung mit Essigsäureanhybrid leicht, quantitativ sowie preiswert zugängliche Isosorbid-2,5-diacetat (IV, R=$CH_3$). Grundsätzlich eignen sich alle Isosorbid-2,5-diester als Ausgangsmaterialien für die unten beschriebenen Hydrolysen und Alkoholysen. Bevorzugt verwendet wird das Diacetat aus Kostengründen sowie wegen des niedrigen Molekulargewichtes der daraus resultierenden

Verbindungen. In allen Fällen handelt es sich bei den Estergruppen um temporäre Schutzgruppen, die für die Herstellung der Nitrate wieder abgespalten werden müssen.
Die enzymatische Hydrolyse von Isosorbid-2,5-diacetat (IV, R = $CH_3$) [2,5-Di-O-acetyl-1,4:3,6-dianhydro-D-sorbit] wird in wässrigem Phosphatpuffer bei pH 7 in Gegenwart einer Hydrolase durchgeführt. Während der enzymatischen Hydrolyse sinkt der pH-Wert deutlich ab und wird durch kontinuierliche Zugabe von 1 molarer Natronlauge mittels eines Autotitrators (alternativ einer Schlauchpumpe) konstant auf pH 7 gehalten.
Da das eingesetzte Diacetat fest ist, verläuft diese Zweiphasenreaktion in der beschriebenen Weise zwar ab, aber nur recht langsam. Es wurde nun überraschend gefunden, daß die Zugabe geringer Mengen von THF zum Reaktionsmedium zu einer beträchtlichen Beschleunigung der Reaktion führt.
Die enzymatische Umsetzung verläuft mit äußerst hoher Selektivität, es wird ausschließlich die Acetatfunktion an der

C(5) - Position hydrolysiert. Man erhält so in nahezu quantitativer Ausbeute (>90% isoliert) isomerenreines Isosorbid-2-acetat (II, R = $CH_3$) (vgl. Abb. 1).
Alle Hydrolasen sind - hohe Selektivität vorausgesetzt - grundsätzlich zur Durchführung dieser enzymatischen Hydrolysen geeignet. Bevorzugt werden kommerziell verfügbare Lipasen der Stämme *Pseudomonas* oder *Mucor miehei*, wobei sich die kommerziell verfügbaren Lipasen aus *Pseudomonas fluorescens* (Amano Pharmaceuticals Co., Boehringer Mannheim) besonders bewährt haben.
Die Menge an benötigtem Enzym richtet sich nach der hydrolytischen Aktivität des Biokatalysators. Das jeweils beste Verhältnis von Substrat und Enzym muß unter den spezifischen Reaktionsbedingungen des Experimentes empirisch bestimmt werden. Anzustreben ist eine möglichst hohe Raum-Zeit-Ausbeute.
Die Reaktionen werden bei pH 5 - 9 durchgeführt. Bevorzugt ist pH 6 - 8, besonders bevorzugt pH 7.
Üblicherweise werden derartige Reaktionen in Zweiphasen-Systemen, bestehend aus dem festen oder flüssigen Substrat sowie einem wässrigen Puffer im Verhältnis 1 : 10 bis 10 : 1 durchgeführt. Besonders bevorzugt ist ein Verhältnis von 1:10 bis 1 : 2.
Derartige Hydrolysen werden zu besseren Konstanthaltung des pH-Wertes gewöhnlich in Gegenwart von Puffern durchgeführt. Ein Puffer ist aber keine notwendige Voraussetzung für eine erfolgreiche Durchführung des Verfahrens. Enzymatische Hydrolysen können auch in salzarmen, wässrigen Lösungen durchgeführt werden. Zur Konstanthaltung des pH-Wertes können auch andere Basen, wie z.B. Ammoniak verwendet werden.
Wie oben beschrieben, erweist sich der Zusatz von Cosolventien (z.B. THF, Aceton etc.) als essentiell für den Erfolg des Verfahrens.
Derartige enzymatische Hydrolysen werden gewöhnlich bei Temperaturen zwischen 0 - 70°C durchgeführt. Besonders bevorzugt ist dabei die Durchführung im Temperatur-Optimum des Enzyms bei gewöhnlich 30 - 60°C, aber deutlich unterhalb des Temperatur-Maximums von ca. 65°C. Die Reaktionen lassen sich aber auch schon bei Raumtemperatur durchführen. Oft wird im Labor dieser Bereich aus Gründen der Bequemlichkeit gewählt.
Grundsätzlich sind alle Isosorbid-2,5-diester mit Fettsäureresten der Kettenlängen von C-1 bis C-18 zur Durchführung des Verfahrens geeignet. Es handelt sich ja in allen Fällen um die natürlichen Substrate der verwendeten Biokatalysatoren. Wie bereits oben erwähnt, wurden die Diacetate aus praktischen und ökonomischen Erwägungen bevorzugt eingesetzt.
Das bei den selektiven enzymatischen Hydrolysen ausschließlich gebildete Isosorbid-2-acetat (II, R = $CH_3$) wird aus dem Reaktionsgemisch durch einfache Extraktion isoliert und kristallisiert nach Entfernung des Lösungsmittels in reiner Form aus.

Alternativ zur enzymatischen Hydrolyse kann die gleiche Transformation auch durch enzymatische Alkoholyse erreicht werden. Bei diesem Verfahren wird das wässrige Zweiphasen-System durch ein homogenes, organisches Reaktionsmedium ersetzt. Anstelle von Wasser wird eine Alkohol als nucleophile Reaktionskomponente verwendet. Dazu wird Isosorbid-2,5-diacetat (IV, R = $CH_3$) in Aceton gelöst, mit einem Überschuß an n-Heptanol versetzt und die Mischung bis zur vollständigen Abspaltung einer Estergruppe gerührt.
Wiederum wird ausschließlich die Estergruppe an C(5) abgespalten, praktisch quantitativ wird dabei Isosorbid-2-acetat erhalten.
Die oben aufgeführten Argumente bezüglich der verwendeten Enzyme, ausgewählten Reaktionsbedingungen sowie der verwendeten Isosorbid-2,5-diester als Ausgangsmaterialien gelten identisch.
Grundsätzlich sind alle organischen Lösungsmittel als Reaktionsmedien geeignet, sofern die eingesetzten Hydrolasen darin noch eine ausreichende Aktivität besitzen. Dies muß, genau wie das optimale Verhältnis von Substrat und Enzym unter den spezifischen Reaktionsbedingungen am besten empirisch bestimmt werden.
Grundsätzlich eignen sich alle Alkohole mit ausreichender Nucleophilie zur Durchführung derartiger enzymatischer Alkoholysen. Aus praktischen Gründen, insbesondere zur einfachen Aufarbeitung und Isolation der Produkte werden bevorzugt Alkohole gewählt deren Ester (z.B. Heptylacetat) leicht destillativ abgetrennt werden können.

Alternativ zu den oben beschriebenen Hydrolysen und Alkoholysen von Isosorbid-2,5-diestern gelingt auch die enzymatische Veresterung von Isosorbid (III) und zwar mit ebenso hoher Selektivität. Dazu wird Isosorbid in Aceton gelöst und wiederum in Gegenwart von Hydrolasen unter Verwendung von Vinylacetat als Acyldonor acyliert. Die enzymatische Veresterung verläuft wiederum mit extrem hoher Selektivität und spezifisch an der 5-Position von Isosorbid. Man erhält dabei in einer praktisch quantitativen Reaktion ausschließlich Isosorbid-5-acetat (I, R = $CH_3$) (vgl. Abb. 2).

Alle organischen Lösungsmittel sind grundsätzlich als Reaktionsmedien geeignet, soweit sie nicht selbst verestert werden können, wie z.B. Alkohole. Ihre Eignung muß unter den spezifischen Reaktionsbedingungen am besten empirisch bestimmt werden.
Alle Acyldonatoren (Carbonsäuren, Carbonsäureester, Carbonsäureanhydride, Glyceride, Alkenylester etc.) sind grundsätzlich zur Durchführung derartiger enzymatischer Veresterungen geeignet. Bevorzugt sind Vinylester, da diese die Durchführung der enzymatischen Veresterungen unter den Bedingungen des irreversiblen Acyltransfers gestatten. Der dabei freigesetzte Vinylalkohol tautomerisiert spontan zum gasförmigen Acetaldehyd, der damit dem Reaktionsgleichgewicht irreversibel entzogen wird. Alle Vinylester mit Fettsäurekettenlängen von C1-C18 sind grundsätzlich dafür geeignet. Besonders bevorzugt wird Vinylacetat als preiswerter Acyldonor.
Alle Hydrolasen sind grundsätzlich zur Durchführung enzymatischer Veresterungen geeignet, vorausgesetzt, sie zeigen die geforderte hohe Selektivität. Bevorzugt sind wiederum kommerziell verfügbare Lipasen der Stämme *Pseudomonas fluorescens* (Amano Pharmaceutical Co., Boehringer Mannheim). Die Veresterung gelingt auch mit anderen Hydrolasen, z.B. einer Lipase aus *Mucor miehei*. Wiederum findet man eine hohe Selektivität, jedoch ist die Reaktion sehr langsam, die Umsetzung bleibt unvollständig und macht dadurch ein Trennverfahren zur Isolation der Produkte erforderlich.

Aufgrund der extrem hohen Selektivitäten, der komplementären Bildung beider Stereoisomerer und der einfachen Durchführbarkeit der Reaktionen sind bei dem beschriebenen Verfahren alle wesentlichen Voraussetzungen für eine erfolgreiche technische Anwendung der Methode erfüllt.
Beide Isomere, Isosorbid-5-acetat (I, R = $CH_3$) und Isosorbid-2-acetat (II, R = $CH_3$) können damit wahlweise, ohne aufwendige Reinigungsschritte und praktisch quantitativ in isomerenreiner Form gewonnen werden. Sie stehen damit als auch als Ausgangsmaterialien zur Überführung in das entsprechende Isosorbid-2-nitrat (V) sowie Isosorbid-5-nitrat (VI) zur Verfügung.
Dazu wird z.B. Isosorbid-2-acetat zunächst mit Salpetersäure in Eisessig oder Essigsäureanhydrid nitriert und danach die Acetatfunktion mit Kaliumcarbonat in Methanol abgespalten. Man erhält dabei Isosorbid-5-nitrat in isomerenreiner Form und in vorzüglicher Ausbeute (Abb. 1).
Auf analoge Weise kann Isosorbid-5-acetat in Isosorbid-2-nitrat überführt werden (Abb. 2). Die Eigenschaften aller hergestellten Produkte sind in Tab. 1 zusammengefasst.

Durch die überraschend hohe Selektivität enzymatischer Reaktionsschritte ist ein äußerst einfacher Zugang zu diesen pharmazeutisch interessanten Verbindungen erschlossen worden, der sich problemlos in den technischen Maßstab übertragen lassen sollte.

Die Erfindung wird in den folgenden Beispielen weiter erläutert.

Tabelle 1:

Isosorbid-2-acetat: farblose, kristalline Substanz: Schmp. 75 - 76°C
DC (Diethylether): $R_f$ = 0.28; $[\alpha]_D^{20}$ = + 77.1° (c = 1.12, Ethylacetat).
Isosorbid-5-acetat: farblose, viskose Flüssigkeit
DC (Diethylether): $R_f$ = 0.30; $[\alpha]_D^{20}$ = + 117.9° (c = 1.36, Ethylacetat)
Isosorbid-2-nitrat: farblose, kristalline Substanz; Schmp. 52 - 53°C
DC (Hexan - Diethylether 1 - 1): $R_f$ = 0.11
Isosorbid-5-nitrat: farblose, kristalline Substanz; Schmp. 88°C
DC (Hexan - Diethylether 1 - 1): $R_f$ = 0.10; $[\alpha]_D^{20}$ = + 181° (c = 0.99, Ethylacetat)
2-O-Acetyl-isosorbid-5-nitrat: farblose kristalline Substanz; Schmp. 95 - 96°C
DC (Hexan - Diethylether 1 - 1): $R_f$ = 0.36; $[\alpha]_D^{20}$ = + 163° (c = 1.00, Ethylacetat)
5-O-Acetyl-isosorbid-2-nitrat: farblose, hochviskose Flüssigkeit
DC (Hexan - Diethylether 1 - 1): $R_f$ = 0.38

**Isosorbid-2-acetat [1,4:3,6-Dianhydro-D-sorbit-2-acetat]**

Beispiel 1:

2.64 g (11.5 mmol) Isosorbid-2,5-diacetat werden in 20 ml Phosphatpuffer pH 7.0 suspendiert und mit 200 mg Lipase aus *Pseudomonas fluorescens* (SAM II, Amano Pharmaceuticals) versetzt. Der pH-Wert wird mit Hilfe einer Autobürette durch zudosieren von 1M Natronlauge konstant gehalten. Nach einem Verbrauch von 10.95 ml 1M Natronlauge (40 Stunden) extrahiert man den Ansatz kontinuierlich für 24 Stunden mit Ethylacetat. Die organische Phase trocknet man über $MgSO_4$ und zieht das Lösungsmittel ab. Das Rohprodukt trennt man säulenchromatographisch (Kieselgel 60) in Diethylether.

Ausbeute: 494 mg (3.38 mmol) Isosorbid (29%)
farblose kristalline Substanz
Schmp. 61 - 62°C
180 mg (0.96 mmol) Isosorbid-2-acetat (8%)
farblose kristalline Substanz
Schmp. 70 - 71°C

1070 mg (4.65 mmol) Isosorbid-2,5-diacetat (40%)
farblose hochviskose Flüssigkeit

Beispiel 2:

2.30 g (10 mmol) Isosorbid-2,5-diacetat werden in 5 ml abs. THF gelöst, mit 20 ml Phosphatpuffer pH 7.0 sowie 400 mg Lipase aus *Pseudomonas fluorescens* (SAM II, Amano Pharmaceuticals) versetzt und nach einem Verbrauch von 10.03 ml 1M Natronlauge (48 Stunden) analog zu Beispiel 1 aufgearbeitet.

Ausbeute: 1.60 g (8.50 mmol) Isosorbid-2-acetat (85%)
farblose kristalline Substanz
Schmp. 72 - 73°C

Beispiel 3:

6.91 g (30 mmol) Isosorbid-2,5-diacetat werden in 15 ml abs. THF gelöst, mit 60 ml Phosphatpuffer pH 7.0 sowie 500 mg Lipase aus *Pseudomonas fluorescens* (SAM II, Amano Pharmaceuticals) versetzt und nach einem Verbrauch von 29.07 ml 1M Natronlauge (24 Stunden) analog zu Beispiel 1 aufgearbeitet.

Ausbeute: 5.00 g (26.7 mmol) Isosorbid-2-acetat (89%)
farblose kristalline Substanz
Schmp. 75 - 76°C

Beispiel 4:

2.30 g (10 mmol) Isosorbid-2,5-diacetat werden in 15 ml Aceton gelöst, mit 5ml n-Heptanol sowie 200 mg Lipase aus *Pseudomonas fluorescens* (SAM II, Amano Pharmaceuticals) versetzt und bei Raumtemperatur gerührt. Nach 12 Tagen filtriert man das Enzym ab, wäscht es gründlich mit Aceton und destilliert das Lösungsmittel sowie den Überschuß an n-Heptanol sowie das entstandene Heptylacetat ab.

Ausbeute: 1.71 g (9.09 mmol) Isosorbid-2-acetat (91%)
farblose kristalline Substanz
Schmp. 75 - 76°C

**Isosorbid-5-acetat [1,4:3,6-Dianhydro-D-sorbit-5-acetat]**

Beispiel 5:

1.46 g (10 mmol) Isosorbid werden in 20 ml Aceton gelöst, mit 2.77 ml (30 mmol) Vinylacetat sowie 200 mg Lipase aus *Pseudomonas fluorescens* (SAM II, Amano Pharmaceuticals) versetzt und bei Raumtemperatur gerührt. Nach 3 Tagen filtriert man das Enzym ab, wäscht es gründlich mit Aceton und zieht das Lösungsmittel ab. Das Rohprodukt destilliert man am Kugelrohr.

Ausbeute: 1.70 g (9.03 mmol) Isosorbid-5-acetat (90%)
farblose viskose Flüssigkeit
Sdp. 125°C/0.08 mbar

Beispiel 6:

14.61 g (100 mmol) Isosorbid werden in 150 ml Aceton gelöst, mit 22 ml (300 mmol) Vinylacetat sowie 500 mg Lipase SAM II versetzt und 6 Tage bei Raumtemperatur gerührt. Anschließend filtriert man das Enzym ab, wäscht es mit Aceton, zieht das Lösungsmittel ab und destilliert das Rohprodukt im Hochvakuum.

Ausbeute: 16.90 g (89.9 mmol) Isosorbid-5-acetat (90%)
farblose viskose Flüssigkeit
Sdp. 130°C/0.09 mbar

**Isosorbid-2-nitrat [1,4:3,6-Dianhydro-D-sorbit-2-nitrat]**

Beispiel 7:

0.2 ml 65%ige Salpetersäure werden bei 0°C mit 0.8 ml Essigsäureanhydrid gemischt und bei dieser Temperatur tropfenweise mit einer Lösung aus 0.2 ml Dichlormethan und 330 mg (1.75 mmol) Isosorbid-5-acetat versetzt. Man rührt noch 20 Minuten bei Raumtemperatur nach, setzt 0.4 ml Dichlormethan und 1 ml Wasser zu und trennt die Phasen. Die organische Phase wird solange gegen verd. Ammoniaklösung extrahiert bis sie neutral ist, man trocknet sie über $MgSO_4$ und zieht das Lösungsmittel ab.

Ausbeute: 400 mg (1.7 mmol) 5-O-Acetyl-isosorbid-2-nitrat (98%)
farblose, hochviskose Flüssigkeit

400 mg (1.7 mmol) 5-O-Acetyl-isosorbid-2-nitrat werden zu 10 ml Methanol sowie 50 mg Kaliumcarbonat gegeben und die Mischung für 12 Stunden bei Raumtemperatur gerührt. Anschließend zieht man das Lösungsmittel ab, nimmt den Rückstand in 2 ml Wasser auf und extrahiert 4 mal mit je 10 ml Ethylacetat. Die vereinigte organische Phase wird vom Lösungsmittel befreit und der Rückstand im Hochvakuum getrocknet.

Ausbeute: 300 mg (1.56 mmol) Isosorbid-2-nitrat (89%)
farblose kristalline Substanz
Schmp. 52 - 53°C

**Isosorbid-5-nitrat [1,4:3,6-Dianhydro-D-sorbit-5-nitrat]**

Beispiel 8:

1.4 ml 65%ige Salpetersäure werden bei 0°C mit 5.5 ml Essigsäureanhydrid gemischt und bei dieser Temperatur tropfenweise mit einer Lösung aus 1.4 ml Dichlormethan und 2.6 g (13.8 mmol) Isosorbid-2-acetat versetzt. Man rührt noch 20 Minuten bei Raumtemperatur nach, setzt 2.8 ml Dichlormethan und 6.9 ml Wasser zu und trennt die Phasen. Die organische Phase wird solange gegen verd. Ammoniaklösung extrahiert bis sie neutral ist, man trocknet sie über $MgSO_4$ und zieht das Lösungsmittel ab.

Ausbeute: 2.73 g (11.7 mmol) 2-O-Acetyl-isosorbid-5-nitrat (85%)
farblose kristalline Substanz
Schmp. 95 - 96°C

800 mg (3.4 mmol) 2-O-Acetyl-isosorbid-5-nitrat werden zu 20 ml Methanol sowie 100 mg Kaliumcarbonat gegeben und die Mischung für 14 Stunden bei Raumtemperatur gerührt. Anschließend zieht man das Lösungsmittel ab, nimmt den Rückstand in 20 ml Wasser auf und extrahiert 6 mal mit je 20 ml Ethylacetat. Die vereinigte organische Phase wird vom Lösungsmittel befreit und der Rückstand im Hochvakuum getrocknet.

Ausbeute: 590 mg (3.1 mmol) Isosorbid-5-nitrat (91%)
farblose kristalline Substanz
Schmp. 88°C

**Patentansprüche**

**1.** Verfahren zur Herstellung isomerenreiner Isosorbid-2-monoester der allgemeinen Formel II, dadurch gekennzeichnet, daß man Isosorbid-2,5-diester der allgemeinen Formel IV,

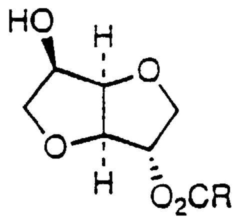

II

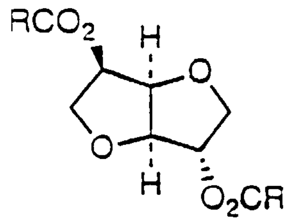

IV

wobei R = H und Alkyl von C1 bis C17 sowie halogeniertes Alkyl bedeutet, mit einer Hydrolase im pH-Bereich 5 - 9 umsetzt und dabei den isomerenreinen Isosorbid-2-monoester der allgemeinen Formel II gewinnt.

**2.** Verfahren nach Anspruch 1, dadurch gekennzeichnet, daß man als Substrat Isosorbid-2,5-diacetat (IV; R = $CH_3$) verwendet.

**3.** Verfahren nach Anspruch 1 und 2, dadurch gekennzeichnet, daß man dem wässrigen Reaktionsmedium ein Cosolvents zusetzt.

**4.** Verfahren nach Ansprüchen 1 bis 3, dadurch gekennzeichnet, daß man als Cosolvents Tetrahydrofuran zusetzt.

**5.** Verfahren nach Ansprüchen 1 - 4, dadurch gekennzeichnet, daß man als Hydrolasen Lipasen aus *Pseudomonas fluorescens* verwendet.

**6.** Verfahren zur Herstellung isomerenreiner Isosorbid-2-monoester der allgemeinen Formel II, dadurch gekennzeichnet, daß man Isosorbid-2,5-diester der allgemeinen Formel IV, gelöst in einem organischen Lösungsmittel und in Gegenwart eines Alkohols mit einer Hydrolase inkubiert und dabei den isomerenreinen Isosorbid-2-monoester der allgemeinen Formel II gewinnt.

**7.** Verfahren nach Anspruch 6, dadurch gekennzeichnet, daß man als Substrat Isosorbid-2,5-diacetat (IV, R = $CH_3$) verwendet.

**8.** Verfahren nach Anspruch 6 bis 7, dadurch gekennzeichnet, daß man als Alkoholkomponente Alkanole der Kettenlänge C-1 bis C-10 verwendet.

**9.** Verfahren nach Ansprüchen 6 - 8, dadurch gekennzeichnet, daß man als Hydrolasen Lipasen aus *Pseudomonas fluorescens* verwendet.

**10.** Verfahren zur Herstellung isomerenreiner Isosorbid-5-monoester der allgemeinen Formel I,

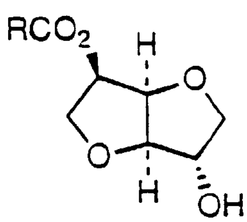

I

wobei R = H und Alkyl von C1 bis C17 sowie halogeniertes Alkyl bedeutet, dadurch gekennzeichnet, daß man Isosorbid (III)

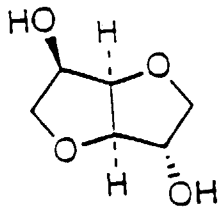

III

, gelöst in einem organischen Lösungsmittel und in Gegenwart einer Hydrolase mit einem Acyldonor umsetzt und dabei den isomerenreinen Isosorbid-5-ester der allgemeinen Formel I isoliert.

**11.** Verfahren nach Anspruch 10, dadurch gekennzeichnet, daß man als Acyldonatoren Carbonsäuren, Carbonsäureester, Carbonsäureanhydride, Glyceride, Alkenylester verwendet.

**12.** Verfahren nach Ansprüchen 10 bis 11, dadurch gekennzeichnet, daß man als Acyldonatoren Vinylester verwendet.

**13.** Verfahren nach Ansprüchen 10 bis 12, dadurch gekennzeichnet, daß man als Acyldonor Vinylacetat verwendet.

**14.** Verfahren nach Ansprüchen 10 bis 13, dadurch gekennzeichnet, daß man als Lösungsmittel Aceton verwendet.

**15.** Verfahren nach Ansprüchen 10 bis 14, dadurch gekennzeichnet, daß man als Hydrolasen Lipasen aus *Pseudomonas fluorescens* verwendet.

**Claims**

**1.** Process for the production of isomerically pure isosorbide-2-monoesters having the general formula II, wherein an isosorbide-2,5-diester having the general formula IV,

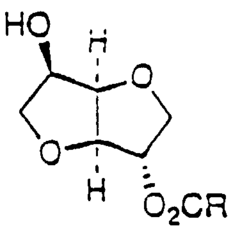

II

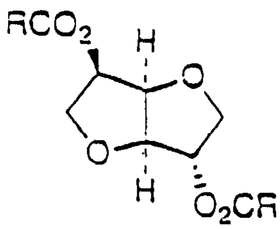

IV

in which R = H and alkyl denotes C1 to C17 as well as halogenated alkyl, is reacted with a hydrolase in the pH range 5 - 9 and in this process the isomerically pure isosorbide-2-monoester having the general formula II is obtained.

**2.** Process as claimed in claim 1, wherein isosorbide-2,5-diacetate (IV; R = $CH_3$) is used as the substrate.

**3.** Process as claimed in claim 1 and 2, wherein a cosolvent is added to the aqueous reaction medium.

**4.** Process as claimed in claims 1 to 3, wherein tetrahydrofuran is added as the cosolvent.

**5.** Process as claimed in claims 1 - 4,. wherein lipases from *Pseudomonas fluorescens* are used as the hydrolases.

**6.** Process for the production of isomerically pure isosorbide-2-monoesters having the general formula II, wherein isosorbide-2,5-diesters having the general formula IV dissolved in an organic solvent are incubated with a hydrolase in the presence of an alcohol and in this process the isomerically pure isosorbide-2-monoester having the general

formula II is obtained.

7. Process as claimed in claim 6, wherein isosorbide-2,5-diacetate (IV, R = $CH_3$) is used as the substrate.

8. Process as claimed in claim 6 to 7, wherein alkanols with a chain lengths of C-1 to C-10 are used as the alcohol component.

9. Process as claimed in claims 6 - 8, wherein lipases from *Pseudomonas fluorescens* are used as the hydrolases.

10. Process for the production of isomerically pure isosorbide-5-monoesters having the general formula I,

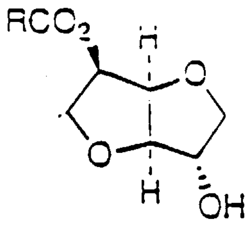

I .

in which R = H and alkyl denotes C1 to C17 as well as halogenated alkyl, wherein isosorbide (III)

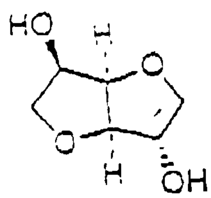

III

dissolved in an organic solvent is reacted with an acyl donor in the presence of a hydrolase and in this process the isomerically pure isosorbide-5-ester having the general formula I is isolated.

11. Process as claimed in claim 10, wherein carboxylic acids, carboxylic acid esters, carboxylic acid anhydrides, glycerides, alkenyl esters are used as the acyl donors.

12. Process as claimed in claims 10 to 11, wherein vinyl esters are used as the acyl donors.

13. Process as claimed in claims 10 to 12, wherein vinyl acetate is used as the acyl donor.

14. Process as claimed in claims 10 to 13, wherein acetone is used as the solvent.

15. Process as claimed in claims 10 to 14, wherein lipases from *Pseudomonas fluorescens* are used as the hydrolases.

**Revendications**

1. Procédé de préparation d'un isomère pur d'un 2-monoester d'isosorbide de formule générale II, caractérisé en ce que l'on fait réagir un 2,5-diester d'isosorbide de formule générale IV

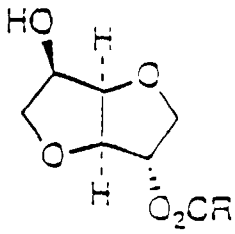

II

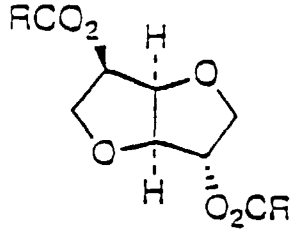

IV

dans laquelle R=H et le groupe alkyle signifie un groupe alkyle en C1 à C17 ou un groupe alkyle halogéné, avec une hydrolase dans un domaine de pH allant de 5 à 9, et l'on obtient ainsi l'isomère pur de 2-monoester d'isosorbide de formule générale II.

2. Procédé selon la revendication 1, caractérisé en ce que l'on utilise, en tant que substrat, le 2,5-diacétate d'isosorbide (IV ; R=$CH_3$).

3. Procédé selon les revendications 1 et 2, caractérisé en ce que l'on ajoute un cosolvant au milieu réactionnel aqueux.

4. Procédé selon les revendications 1 à 3, caractérisé en ce que, en tant que cosolvant, on ajoute du tétrahydrofurane.

5. Procédé selon les revendications 1 à 4, caractérisé en ce que, en tant qu'hydrolase, on utilise des lipases de *Pseudomonas fluorescens.*

6. Procédé de préparation d'un isomère pur d'un 2-monoester d'isosorbide de formule générale II, caractérisé en ce que l'on incube le 2,5-diester d'isosorbide de formule générale IV, en solution dans un solvant organique et en présence d'un alcool, avec une hydrolase et on obtient ainsi l'isomère pur de 2-monoester d'isosorbide de formule générale II.

7. Procédé selon la revendication 6, caractérisé en ce que l'on utilise, en tant que substrat, le 2,5-diacétate d'isosorbide (IV ; R=$CH_3$).

8. Procédé selon les revendications 6 à 7, caractérisé en ce que l'on utilise, en tant que composant alcool, des alcanols ayant une longueur de chaîne allant de C1 à C10.

9. Procédé selon les revendications 6 à 8, caractérisé en ce que l'on utilise, en tant qu'hydrolases, des lipases de *Pseudomonas fluorescens.*

10. Procédé de préparation d'un isomère pur de 2-monoester d'isosorbide de formule générale I,

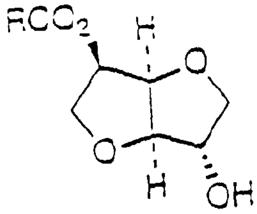

I

dans laquelle R=H et le groupe alkyle signifie un groupe alkyle en C1 à C17 ou un groupe alkyle halogéné, caractérisé en ce que l'on fait réagir l'isosorbide III,

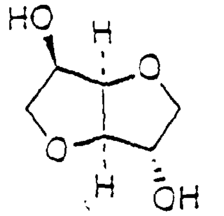

III

en solution dans un solvant organique et en présence d'une hydrolase, avec un donneur d'acyle et on isole ainsi l'isomère pur de 5-ester d'isosorbide de formule générale I.

**11.** Procédé selon la revendication 10, caractérisé en ce que, en tant que donneur d'acyle, on utilise des acides carboxyliques, des esters d'acides carboxyliques, des anhydrides d'acides carboxyliques, des glycérides, des esters d'alcényle.

**12.** Procédé selon les revendications 10 à 11, caractérisé en ce que, en tant que donneur d'acyle, on utilise un ester vinylique.

**13.** Procédé selon les revendications 10 à 12, caractérisé en ce que, en tant que donneur d'acyle, on utilise de l'acétate de vinyle.

**14.** Procédé selon les revendications 10 à 13, caractérisé en ce que, en tant que solvant, on utilise de l'acétone.

**15.** Procédé selon les revendications 10 à 14, caractérisé en ce que, en tant qu'hydrolase, on utilise des lipases de *Pseudomonas fluorescens.*

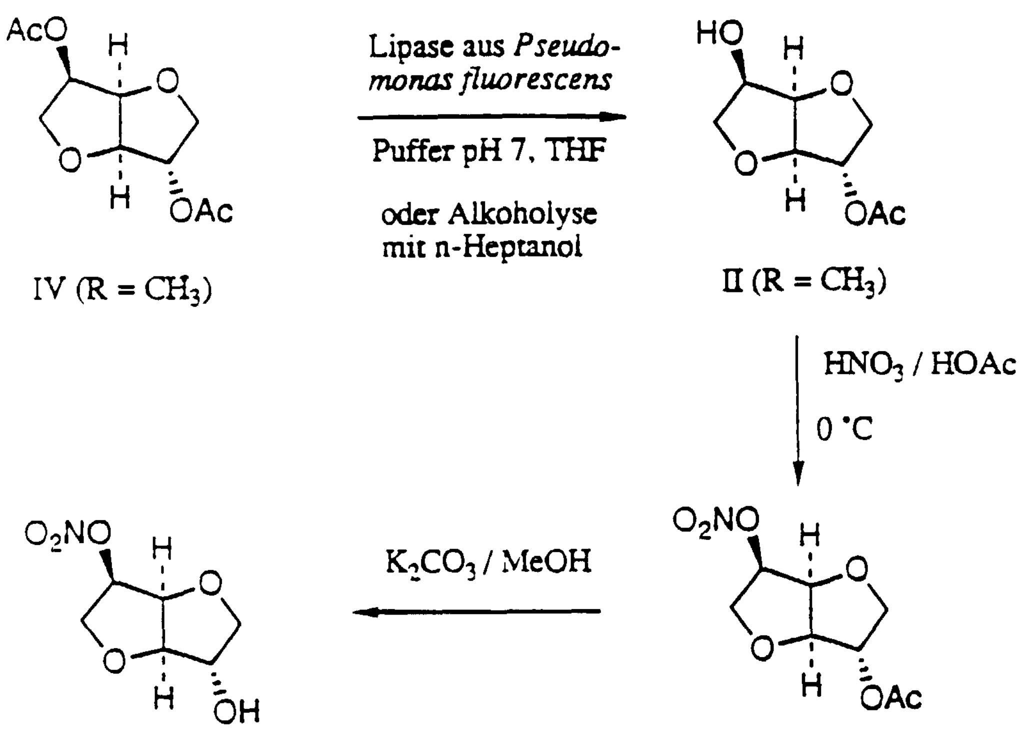


IV (R = $CH_3$)

II (R = $CH_3$)

VI

Abb.1. Chemoenzymatische Herstellung von Isosorbid-5-nitrat (VI).

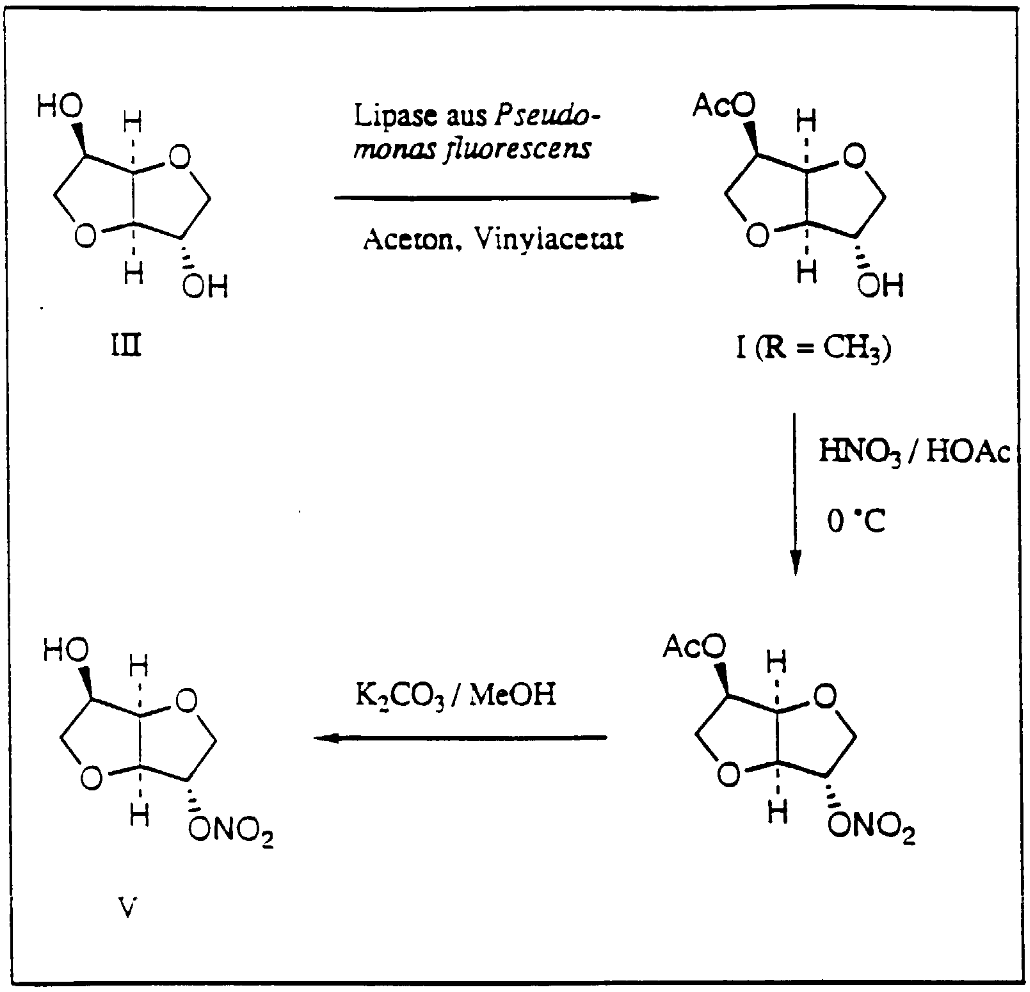


Abb. 2. Chemoenzymatische Herstellung von Isosorbid-2-nitrat (V)